# EUROPEAN PATENT SPECIFICATION

(11) **EP 3 840 655 B1**
(45) Date of publication and mention of the grant of the patent: **09.10.2024**
(21) Application number: 19755330.8
(22) Date of filing: 13.08.2019
(51) Int. Cl.: A61B 8/00, A61B 8/08, A61B 34/20, A61B 90/00

(54) **CONSTRAINING SENSOR TRACKING ESTIMATES IN INTERVENTIONAL ACOUSTIC IMAGING**
EINSCHRÄNKUNG VON SENSORVERFOLGUNGSSCHÄTZUNGEN BEI INTERVENTIONELLEN AKUSTISCHEN BILDERN
CONTRAINDRE DES ESTIMATIONS DE SUIVI DE CAPTEUR DANS L'IMAGERIE ACOUSTIQUE INTERVENTIONNELLE

(30) Priority: 22.08.2018 US 201862721173 P
(43) Date of publication of application: 30.06.2021
(73) Proprietor: Koninklijke Philips N.V., 5656 AG Eindhoven (NL)
(72) Inventor: CHEN, Alvin, 5656 AE Eindhoven (NL); BHARAT, Shyam, 5656 AE Eindhoven (NL); JAIN, Ameet, Kumar, 5656 AE Eindhoven (NL); VAIDYA, Kunal, 5656 AE Eindhoven (NL); ERKAMP, Ramon, Quido, 5656 AE Eindhoven (NL); VIGNON, Francois, Guy, Gerard, Marie, 5656AE Eindhoven (NL)
(74) Representative: Philips Intellectual Property & Standards
(86) International application number: PCT/EP2019/071653
(87) International publication number: WO 2020/038766

(56) References cited:
- WO-A1-2018/116114
- US-A1- 2009 088 628
- US-A1- 2009 177 089
- US-A1- 2012 078 103
- US-A1- 2016 317 119
- US-A1- 2016 324 584
- US-A1- 2016 367 216
- ZHAO YUE ET AL: "Evaluation and comparison of current biopsy needle localization and tracking methods using 3D ultrasound", ULTRASONICS, IPC SCIENCE AND TECHNOLOGY PRESS LTD. GUILDFORD, GB, vol. 73, 13 September 2016 (2016-09-13), pages 206 - 220, XP029766201, ISSN: 0041-624X, DOI: 10.1016/J.ULTRAS.2016.09.006

## Description

### TECHNICAL FIELD

This invention pertains to acoustic (e.g., ultrasound) imaging, and in particular a system and computer program for constraining sensor tracking estimates for acoustic imaging in conjunction with an interventional procedure.

### BACKGROUND AND SUMMARY

Acoustic (e.g., ultrasound) imaging systems are increasingly being employed in a variety of applications and contexts. For example, ultrasound imaging is being increasingly employed in the context of ultrasound-guided medical procedures.

Typically, in ultrasound-guided medical procedures the physician visually locates the current position of the needle tip (or catheter tip) in acoustic images which are displayed on a display screen or monitor. Furthermore, a physician may visually locate the current position of the needle on a display screen or monitor when performing other medical procedures. The needle tip generally appears as bright spot in the image on the display screen, facilitating its identification.

However, visualization of an interventional device, or devices, (e.g., surgical instrument(s), needle(s), catheter(s), etc.) employed in these procedures using existing acoustic probes and imaging systems is challenging in many cases. It has been shown that acoustic images may contain a number of artifacts caused by both within-plane (axial and lateral beam axes) and orthogonal-to-the-plane (elevation beam width) acoustic beam formation and it can be difficult to distinguish these artifacts from the device whose position is of interest.

To address these problems, special interventional devices, such as echogenic needles, with enhanced visibility are successfully on the market and provide some improvement at moderate extra cost.

However, due to noise, false echoes, and various other factors, consistently correct identification of the location of the interventional device in acoustic images remains a problem.

Documents US2016/317119A1, US2012/078103A1 disclose known ultrasound imaging systems for visualization and localisation of an intervention device.

Accordingly, it would be desirable to provide an ultrasound system and a method which can provide enhanced acoustic imaging capabilities during interventional procedures. In particular it would be desirable to provide an ultrasound system and a method which can provide improved device tracking estimates during an interventional procedure.

In one aspect of the invention, An acoustic imaging instrument connectable to an acoustic probe having an array of acoustic transducer elements, the acoustic imaging instrument, when connected to the acoustic probe, configured to provide transmit signals to least some of the acoustic transducer elements to cause the array of acoustic transducer elements to transmit an acoustic probe signal to an area of interest, and further configured to produce acoustic images of the area of interest in response to acoustic echoes received from the area of interest in response to the acoustic probe signal, the acoustic imaging instrument including:
- a receiver interface connectable to an intervention device and configured to receive one or more sensor signals from at least one passive sensor disposed on a surface of the intervention device when the intervention device is connected to the receiver interface and the at least one passive sensor is disposed in the area of interest, the one or more sensor signals having been produced in response to the acoustic probe signal; and
- a processor configured to ascertain, from the one or more sensor signals from the passive sensor, an estimated location of the passive sensor in the area of interest, by:
- identifying one or more candidate locations for the passive sensor based on localized intensity peaks in sensor data produced in response to the one or more sensor signals from the passive sensor, and
- using intra-procedural context-specific information to identify the candidate location of the one or more candidate locations which best matches the intra-procedural context-specific information as the estimated location of the passive sensor,
   wherein the processor is further configured to cause a display device to display acoustic images of the area of interest and a marker in the acoustic images to indicate the estimated location.

In another aspect of the invention, a system comprises: an acoustic probe having an array of acoustic transducer elements; and an acoustic imaging instrument connected to the acoustic probe. The acoustic imaging instrument is configured to provide transmit signals to least some of the acoustic transducer elements to cause the array of acoustic transducer elements to transmit an acoustic probe signal to an area of interest, and is further configured to produce acoustic images of the area of interest in response to acoustic echoes received from the area of interest in response to the acoustic probe signal. The acoustic imaging instrument includes: a display device configured to display the acoustic images; a receiver interface configured to receive one or more sensor signals from at least one passive sensor disposed on a surface of an intervention device disposed in the area of interest, the one or more sensor signals being produced in response to the acoustic probe signal; and a processor. The processor is configured to ascertain, from the one or more sensor signals from the passive sensor, an estimated location of the passive sensor in the area of interest, by: identifying one or more candidate locations for the passive sensor based on localized intensity peaks in sensor data produced in response to the one or more sensor signals from the passive sensor, and using intra-procedural context-specific information to identify a one of the candidate locations which best matches the intra-procedural context-specific information as the estimated location of the passive sensor. The display device displays a marker in the acoustic images to indicate the estimated location of the passive sensor.

In some embodiments, the intra-procedural context-specific information includes at least one of: information identifying an anatomical structure where the sensor is expected to be located; information identifying a likely location of the intervention device in the acoustic images; and information identifying previous estimated locations of the sensor in previous ones of the acoustic images.

In some versions of these embodiments, the intra-procedural context-specific information includes the information identifying the anatomical structure where the sensor is expected to be located, and wherein the processor is configured to execute a region detection or segmentation algorithm to identify the anatomical structure where the sensor is expected to be located in the acoustic images.

In some versions of these embodiments, the intra-procedural context-specific information includes the information identifying the anatomical structure where the sensor is expected to be located, wherein the acoustic imaging instrument is configured to produce color Doppler images of the area of interest in response to one or more receive signals received from the acoustic probe, and wherein the processor is configured to identify the anatomical structure where the sensor is expected to be located by identifying blood flow in the color Doppler images.

In some versions of these embodiments, the intra-procedural context-specific information includes the information identifying a likely location of the intervention device in the acoustic images, and wherein the processor is configured to execute a region detection algorithm or segmentation algorithm to identify the likely location of the intervention device in the acoustic images.

In some versions of these embodiments, the intra-procedural context-specific information includes the information identifying the previous estimated locations of the sensor in previous ones of the acoustic images, and wherein the processor is configured to employ one of a state estimation filter applied to each current candidate location and the previous estimated locations of the sensor; a decomposition of all previous locations of the sensor to identify sensor motion trajectory and compare the sensor motion trajectory to each candidate location; a region of interest (ROI) spatial filter defined around an estimated location of the sensor in a previous frame and applied to each candidate location.

In some embodiments, the intra-procedural context-specific information includes: information identifying an anatomical structure where the sensor is expected to be located; information identifying a likely location of the intervention device in the acoustic images; and information identifying previous estimated locations of the sensor in previous ones of the acoustic images.

In some versions of these embodiments, identifying the one or more candidate locations for the passive sensor based on the localized intensity peaks in the one or more sensor signals at times corresponding to the candidate locations, includes: determining, for each candidate location, a weighted sum or other form of weighted integration of a match between the candidate location and each of: the information identifying the anatomical structure where the sensor is expected to be located; the information identifying the likely location of the intervention device in the acoustic images; and the information identifying the previous estimated locations of the sensor in the previous ones of the acoustic images; and selecting as the estimated location of the passive sensor a one of the candidate locations which has a greatest weighted sum or other form of weighted integration.

In another aspect of the invention, a computer program for performing a method is provided where the method comprises: producing acoustic images of an area of interest in response to one or more receive signals received from an acoustic probe in response to acoustic echoes received by the acoustic probe from the area of interest in response to an acoustic probe signal; receiving one or more sensor signals from a passive sensor disposed on a surface of an intervention device in the area of interest, the one or more sensor signals being produced in response to the acoustic probe signal; identifying one or more candidate locations for the passive sensor based on localized intensity peaks in sensor data produced in response to the one or more sensor signals from the passive sensor; using intra-procedural context-specific information to identify a one of the candidate locations which best matches the intra-procedural context-specific information as an estimated location of the passive sensor; displaying the acoustic images on a display device; and displaying on the display device a marker in the acoustic images to indicate the estimated location of the passive sensor.

In some embodiments, the intra-procedural context-specific information includes at least one of: information identifying an anatomical structure where the sensor is expected to be located; information identifying a likely location of the intervention device in the acoustic images; and information identifying previous estimated locations of the sensor in previous ones of the acoustic images.

In some versions of these embodiments, the intra-procedural context-specific information includes the information identifying the anatomical structure where the sensor is expected to be located, and wherein the method includes executing a region detection algorithm or segmentation algorithm to identify the anatomical structure where the sensor is expected to be located in the acoustic images.

In some versions of these embodiments, the intra-procedural context-specific information includes the information identifying the anatomical structure where the sensor is expected to be located, and the method includes: producing color Doppler images of the area of interest in response to the one or more receive signals received from the acoustic probe; and identifying the anatomical structure where the sensor is expected to be located by identifying blood flow in the color Doppler images.

In some versions of these embodiments, the intra-procedural context-specific information includes the information identifying a likely location of the intervention device in the acoustic images, and wherein the processor is configured to execute a region detection algorithm or segmentation algorithm to identify the likely location of the intervention device in the acoustic images.

In some versions of these embodiments, the intra-procedural context-specific information includes the information identifying the previous estimated locations of the sensor in previous ones of the acoustic images, and the method includes one of: applying a state estimation filter to each current candidate location and the previous estimated locations of the sensor; performing a decomposition of all previous locations of the sensor to identify sensor motion trajectory, and comparing the sensor motion trajectory to each candidate location; and applying a region of interest (ROI) spatial filter, defined around an estimated location of the sensor in a previous frame, to each candidate location.

In some embodiments, the intra-procedural context-specific information includes: information identifying an anatomical structure where the sensor is expected to be located; information identifying a likely location of the intervention device in the acoustic images; and information identifying previous estimated locations of the sensor in previous ones of the acoustic images.

In some versions of these embodiments, identifying the one of the candidate locations which best matches the intra-procedural context-specific information as the estimated location of the passive sensor includes: determining, for each candidate location, a weighted sum or other form of weighted integration of a match between the candidate location and each of the information identifying the anatomical structure where the sensor is expected to be located; the information identifying the likely location of the intervention device in the acoustic images; and the information identifying the previous estimated locations of the sensor in the previous ones of the acoustic images; and selecting as the estimated location of the passive sensor a one of the candidate locations which has a greatest weighted sum or other form of weighted integration.

In yet another aspect of the invention, an acoustic imaging instrument comprises: a receiver interface configured to receive one or more sensor signals from at least one passive sensor disposed on a surface of an intervention device which is disposed in an area of interest; and a processor. The processor is configured to ascertain from the one or more sensor signals an estimated location of the passive sensor in the area of interest, by: identifying one or more candidate locations for the passive sensor based on localized intensity peaks in sensor data produced in response to the one or more sensor signals from the passive sensor, and using intra-procedural context-specific information to identify a one of the candidate locations which best matches the intra-procedural context-specific information as the estimated location of the passive sensor. The processor is further configured to cause a display device to display the acoustic images and a marker in the acoustic images to indicate the estimated location of the passive sensor.

In some embodiments, the intra-procedural context-specific information includes at least one of: information identifying an anatomical structure where the sensor is expected to be located; information identifying a likely location of the intervention device in the acoustic images; and information identifying previous estimated locations of the sensor in previous ones of the acoustic images.

In some embodiments, the intra-procedural context-specific information includes: information identifying an anatomical structure where the sensor is expected to be located; information identifying a likely location of the intervention device in the acoustic images; and information identifying previous estimated locations of the sensor in previous ones of the acoustic images.

In some embodiments, identifying the one of the candidate locations which best matches the intra-procedural context-specific information as the estimated location of the passive sensor includes: determining, for each candidate location, a weighted sum or other means of weighted integration of a match between the candidate location and each of: the information identifying the anatomical structure where the sensor is expected to be located; the information identifying the likely location of the intervention device in the acoustic images; and the information identifying the previous estimated locations of the sensor in the previous ones of the acoustic images; and selecting as the estimated location of the passive sensor a one of the candidate locations which has a greatest weighted sum or other weighted integration.

In some embodiments, determining, for each candidate location, a weighted sum or other means of weighted combination of different information sources, the exact numerical method for combining the information sources, as well as the actual values of the weights, are determined through an empirical optimization. The optimization may be carried out for example on training data specific to the desired application.

In some embodiments, a measure of the certainty or uncertainty of the final output may be additionally provided.

### BRIEF DESCRIPTION OF THE DRAWINGS

FIG. 1 shows one example of an acoustic imaging system, including an acoustic imaging instrument and an acoustic probe.
FIG. 2 illustrates one example embodiment of an interventional device having an acoustic sensor disposed at a distal end thereof.
FIG. 3 illustrates example embodiment of a process of overlaying imaging produced from one or more sensor signals received from an acoustic sensor with an acoustic image produced from an acoustic probe.
FIG. 4 illustrates a process of identifying a location of an acoustic sensor in an acoustic image.
FIG. 5 illustrates an image showing multiple candidate locations of an acoustic sensor based on localized intensity peaks in one or more sensor signals produced by the acoustic sensor at times corresponding to the candidate locations.
FIG. 6 illustrates one example embodiment of a method of improving sensor tracking estimates in interventional acoustic imaging by employing intra-procedural context-specific information.
FIG. 7 illustrates one example embodiment of a method of improving sensor tracking estimates in interventional acoustic imaging by employing anatomical structure constraints.
FIG. 8 illustrates one example embodiment of a method of improving sensor tracking estimates in interventional acoustic imaging by employing constraints based on a structure of a device on which the sensor is provided.
FIG. 9 illustrates one example embodiment of a method of improving sensor tracking estimates in interventional acoustic imaging by employing previous estimated locations of the sensor.
FIG. 10 illustrates graphically an example of improving sensor tracking estimates in interventional acoustic imaging by employing intra-procedural context-specific information.
FIG. 11 illustrates a flowchart of an example embodiment of a method of improving sensor tracking estimates in interventional acoustic imaging by employing intra-procedural context-specific information.
FIG. 12 illustrates a flowchart of an example embodiment of a method of employing anatomical structure constraints to improve sensor tracking estimates in interventional acoustic imaging.
FIG. 13 illustrates a flowchart of an example embodiment of a method of employing constraints based on a structure of a device on which a sensor is provided to improve sensor tracking estimates in interventional acoustic imaging.
FIG. 14 illustrates a flowchart of an example embodiment of a method of employing previous estimated locations of the sensor to improve sensor tracking estimates in interventional acoustic imaging.

### DETAILED DESCRIPTION

The present invention will now be described more fully hereinafter with reference to the accompanying drawings, in which preferred embodiments of the invention are shown. This invention may, however, be embodied in different forms and should not be construed as limited to the embodiments set forth herein. Rather, these embodiments are provided as teaching examples of the invention, where the scope of the invention is defined by the appended claims. Herein, when something is said to be "approximately" or "about" a certain value, it means within 10% of that value.

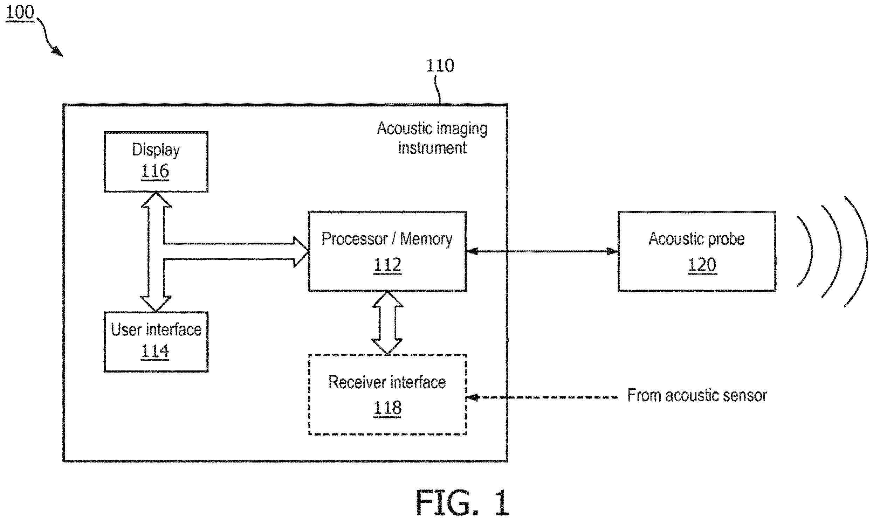

FIG. 1 shows one example of an acoustic imaging system 100 which includes an acoustic imaging instrument 110 and an acoustic probe 120. Acoustic imaging instrument 110 include a processor (and associated memory) 112, a user interface 114, a display device 116 and optionally a receiver interface 118.

In various embodiments, processor 112 may include various combinations of a microprocessor (and associated memory), a digital signal processor, an application specific integrated circuit (ASIC), a field programmable gate array (FPGA), digital circuits and/or analog circuits. Memory (e.g., nonvolatile memory) associated with processor 112 may store therein computer-readable instructions which cause a microprocessor of processor 112 to execute an algorithm to control acoustic imaging system 100 to perform one or more operations or methods which are described in greater detail below. In some embodiments, a microprocessor may execute an operating system. In some embodiments, a microprocessor may execute instructions which present a user of acoustic imaging system 100 with a graphical user interface (GYI) via user interface 114 and display device 116.

In various embodiments, user interface 114 may include any combination of a keyboard, keypad, mouse, trackball, stylus /touch pen, joystick, microphone, speaker, touchscreen, one or more switches, one or more knobs, one or more lights, etc. In some embodiments, a microprocessor of processor 112 may execute a software algorithm which provides voice recognition of a user's commands via a microphone of user interface 114.

Display device 116 may comprise a display screen of any convenient technology (e.g., liquid crystal display). In some embodiments the display screen may be a touchscreen device, also forming part of user interface 114.

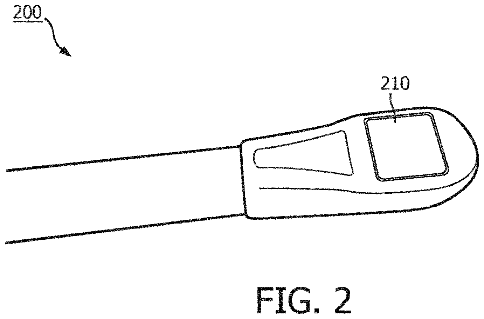

In some embodiments, acoustic imaging instrument 110 may include receiver interface 118 which is configured to receive one or more electrical signals (sensor signals) from an external passive acoustic sensor, for example an acoustic receiver disposed at or near a distal end (tip) of an interventional device, as will be described in greater detail below, particularly with respect to FIG. 2.

Of course it is understood that acoustic imaging instrument 110 may include a number of other elements not shown in FIG. 1, for example a power system for receiving power from AC Mains, an input/output port for communications between processor 112 and acoustic probe 120, a communication subsystem for communicating with other eternal devices and systems (e.g., via a wireless, Ethernet and/or Internet connection), etc.

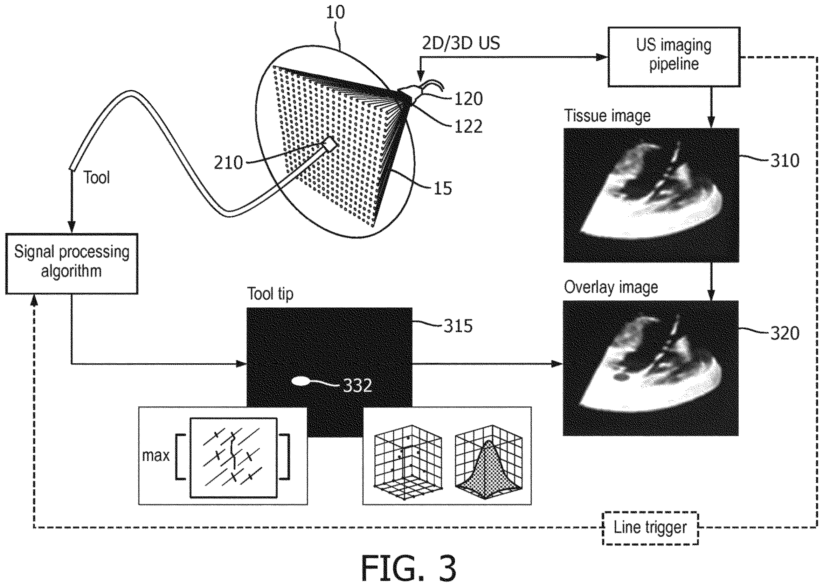

Beneficially, acoustic probe 120 may include an array of acoustic transducer elements 122 (see FIG. 3). At least some of acoustic transducer elements 122 receive transmit signals from acoustic imaging instrument 110 to cause the array of acoustic transducer elements 122 to transmit an acoustic probe signal to an area of interest, and receive acoustic echoes from the area of interest in response to the acoustic probe signal

FIG. 2 illustrates one example embodiment of an interventional device 200 having an acoustic sensor (e.g., a passive acoustic sensor) 210 disposed at a distal end thereof. Although only one passive acoustic sensor 210 is shown for interventional device 200, other embodiments of interventional devices may include two or more passive acoustic sensor(s) 210.

As described in greater detail below, in some embodiments processor 112 of acoustic imaging instrument 110 may use one or more sensor signals received by receiver interface 118 from one or more passive acoustic sensors 210 disposed on interventional device 200 to track the location of interventional device in acoustic images produced from acoustic data produced by echoes received by acoustic probe 120.

In various embodiments, interventional device 200 may comprise a needle, a catheter, a medical instrument, etc.

FIG. 3 illustrates example embodiment of a process of overlaying imaging produced from one or more sensor signals received from an acoustic sensor such as passive acoustic sensor 210 with an acoustic image produced from acoustic echoes received by an acoustic probe such as acoustic probe 120.

As illustrated in FIG. 3, acoustic probe 120 illuminates an area of interest 10 with an acoustic probe signal 15 and receives acoustic echoes received area of interest 10 in response to acoustic probe signal 15. An acoustic imaging instrument (e.g., acoustic imaging instrument 110) produces acoustic images 310 of area of interest 10 in response to acoustic echoes received from area of interest 10 in response to acoustic probe signal 15. In particular, acoustic probe 120 may communicate one or more receive signals (electrical signals) to acoustic imaging instrument 110 in response to acoustic echoes received from area of interest 10 in response to acoustic probe signal 15, and acoustic imaging instrument 110 may produce acoustic images 310 from the receive signal(s).

Meanwhile, a receiver interface (e.g., receiver interface 118) receives one or more sensor signals from at least one passive acoustic sensor (e.g., passive acoustic sensor 210) disposed on a surface of an intervention device (e.g., device 200) disposed in area of interest 10, the one or more sensor signals being produced in response to acoustic probe signal 15. A processor (e.g., processor 112) executes an algorithm to ascertain or determine, from the one or more sensor signals from passive acoustic sensor 210 an estimated location 332 of passive acoustic sensor 210 in area of interest 10. Image 315 illustrates sensor data obtained by processor 112, showing estimated location 332 of passive acoustic sensor 210. For example, processor 112 may employ an algorithm to detect a maximum value or intensity peak in sensor data produced from the one or more sensor signals from passive acoustic sensor 210, and may determine or ascertain that estimated location 332 of passive acoustic sensor 210 corresponds to the location of intensity peak in the sensor data. Then acoustic imaging instrument 110 may overlay the sensor data illustrated in image 315 with acoustic image 310 to produce an overlaid acoustic image 320 which includes a marker to identify estimated location 332 of passive acoustic sensor 210.

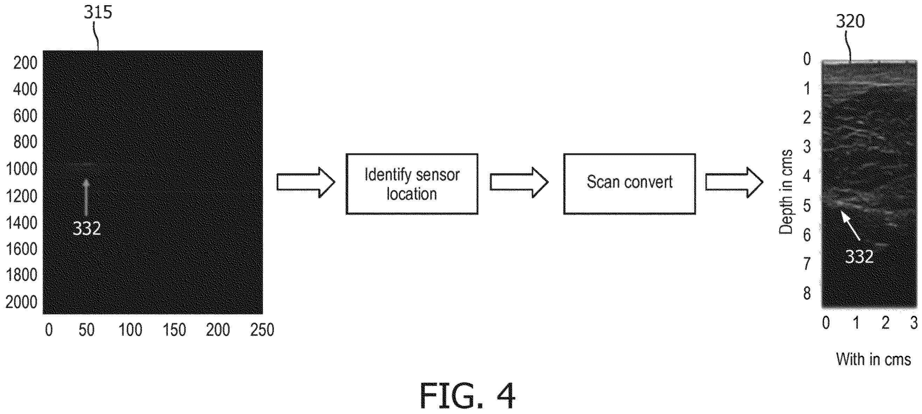

FIG. 4 illustrates a process of identifying an estimated location 332 of passive acoustic sensor 210 in acoustic image 320 when there is only one intensity peak in the sensor data. As shown in FIG. 4, image 315 illustrates sensor data obtained by processor 112 from the sensor signal(s) output by passive acoustic sensor 210, and the single intensity peak is identified as the estimated location 332 of passive acoustic sensor 210. The sensor data is overlaid with the acoustic image data to produce the overlaid acoustic image 320, and a marker is added to indicate estimated location 332 of passive acoustic sensor 210in the overlaid acoustic image 320.

However, as explained above, often the location of passive acoustic sensor 210 in the sensor data is not clear from the sensor data alone. Multiple intensity peaks may occur due to noise and various acoustic aberrations or artifacts. For example, if there is a segment of bone in the imaging plane, an ultrasound beam can bounce off the bone and insonify passive acoustic sensor 210 (an indirect hit), producing a signal that arrives later in time (and that can often be stronger) than the direct insonification. In another example, in tracked needle applications where interventional device 200 is a needle, an ultrasound beam can intersect with the needle shaft and travel down the shaft to passive acoustic sensor 210, resulting in passive acoustic sensor 210 being insonified earlier in time than the direct hit (due to the higher sound speed in the needle shaft compared to that in tissue). In yet another example, random electromagnetic interference (EMI) can cause the system to choose a noise spike as the estimated position of passive acoustic sensor 210.

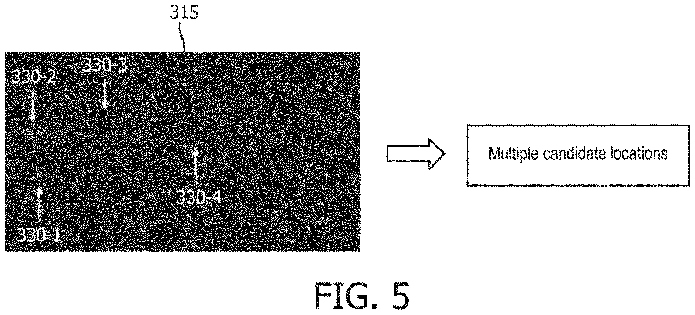

FIG. 5 illustrates an image 315 showing multiple candidate locations (330-1, 330-2, 330-3 and 330-4) of passive acoustic sensor 210 based on localized intensity peaks in one or more sensor signals produced by passive acoustic sensor 210 at times corresponding to the candidate locations.

In this situation, it is not immediately apparent what the best estimated location of passive acoustic sensor 210 is. Indeed, as explained above, it is possible that a "false" intensity peak produced by a reflection or travelling of the shaft of interventional device 200 could be stronger than the intensity peak produced by direct insonification of passive acoustic sensor 210, so simply choosing the greatest intensity peak will often produce a bad estimate for the sensor location.

However, the inventors have appreciated that it is often possible for a processor (e.g., processor 112) of an acoustic imaging instrument and system (e.g., acoustic imaging instrument 110 and acoustic imaging system 100) to identify the best estimated location of a passive acoustic sensor (e.g., passive acoustic sensor 210) disposed on the surface of an interventional device (e.g., interventional device 200), from among a number of candidate locations, during an interventional procedure by factoring into account intra-procedural context-specific information which is available to the processor. Here, intra-procedural context-specific information refers to any data which may be available to the processor pertaining to the context of a specific intervention procedure at the time that the processor is attempting to determine the location of the passive acoustic sensor within the area of interest which is being insonified by the acoustic probe. Such information may include, but is not limited to, the type of interventional device whose sensor is being tracked, known size and/or shape characteristics of the interventional device, known anatomical characteristics within the area of interest where the sensor may be located, a surgical or other procedural plan detailing an expected path for the interventional device and/or sensor to follow within the area of interest during the current intervention procedure; previous known paths, locations, and/or orientations of the interventional device and/or sensor during the current intervention procedure; etc.

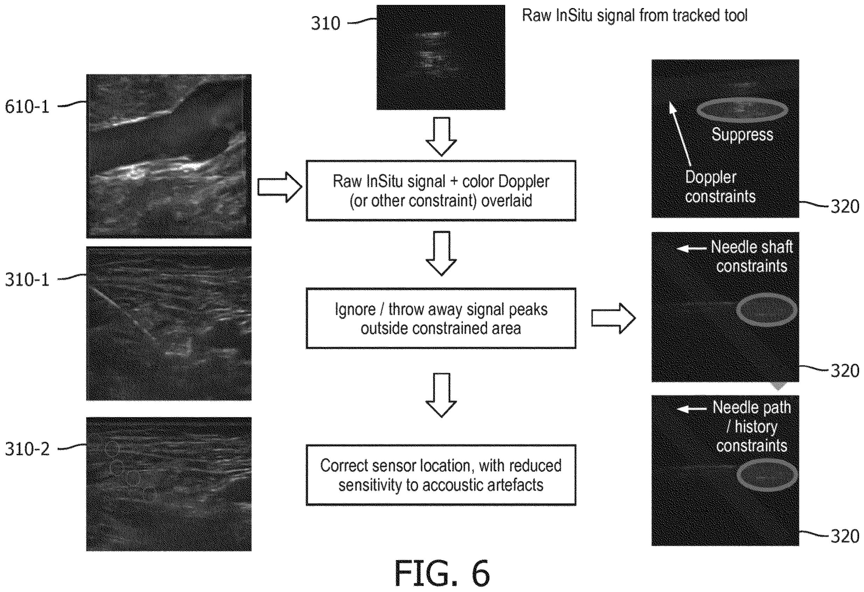

FIG. 6 illustrates one example embodiment of a method of improving sensor tracking estimates in interventional acoustic imaging by employing intra-procedural context-specific information.

FIG. 6 shows an image 315 of sensor data produced in response to one or more sensor signals 15 from passive acoustic sensor 210, as illustrated in FIGs. 1-3 above. One can see several candidate locations for passive acoustic sensor 210, indicated by the bright spots in image 315. Without additional data, it is a difficult if not impossible problem to identify the best estimated location for passive acoustic sensor 210 just from the sensor data of image 315. FIG. 6 illustrates how several different types of intra-procedural context-specific information can be employed as constraints on sensor tracking estimates, eliminating some candidate locations as possibilities and/or selecting one candidate location as the best estimated location.

Consider first the top row of FIG. 6. For endovascular procedures, acoustic imaging system 100 can be operated in Color Doppler mode and the presence of flow is indicative of a blood vessel. Alternately, if acoustic imaging system 100 is operated in B-mode, processor 112 can run segmentation or vessel object detection routines to identify the location and boundaries of the vessel. Since the tracked wire/catheter is being navigated in the vessel, any intensity peaks or "bright spots" in the sensor data matrix that are outside the blood vessel can be considered to be artifacts (except in the rare cases of vessel perforation by a wire/catheter). Processor 112 may employ standard scan conversion routines to convert from B-mode/Color Doppler space to sensor data space, and the intensity peaks or "bright spots" in overlaid acoustic image 320 that are outside the blood vessel can be suppressed or eliminated as possible estimated locations for passive acoustic sensor 210.

Consider next the middle row of FIG. 6. For needle interventions, the estimated sensor location has to be located on the needle shaft. Processor 112 has identified the needle shaft in acoustic image 310-1. This constraint can, thus, be used to weed out incorrect sensor position estimates in overlaid acoustic image 320. Even in cases where the needle shaft is not visible in the acoustic image, the general position and orientation of the needle can be approximately known during the needle insertion. Sensor position estimates that are far away from the approximated needle position and orientation may be weeded out or penalized compared to sensor position estimates that are closer.

Finally, consider the bottom row of FIG. 6. The location of passive acoustic sensor 210 in the current frame or acoustic image 310-2 cannot be inconsistent with history. In other words, if passive acoustic sensor 210 has been progressing smoothly along a certain trajectory, it should not suddenly appear in a totally different location that is not along the path or near the location where it was found in the immediately preceding frame(s) or acoustic image(s). Thus sensor position estimates that are far away from the previous trajectory of the needle may be weeded out or otherwise penalized compared to sensor estimates that are more closely in line with the previous trajectory.

In various embodiments, one or more or all of the intra-procedural context-specific information-based constraints illustrated in the top, middle, and bottom rows of FIG. 6 may be employed to ascertain estimated location 332 of passive acoustic sensor 210. In some embodiments, a weighted combination of constraints may be employed. In particular, in some embodiments, this may include determining, for each candidate location 330 of passive acoustic sensor 210 identified in the sensor data, a weighted sum of matches between the candidate location 330 and each of: information identifying an anatomical structure where passive acoustic sensor 210 is expected to be located; information identifying the likely location of intervention device 200 in acoustic images 320; and information identifying the previous estimated locations 332 of passive acoustic sensor 210 in previous acoustic images 320. The candidate location 330 which has the greatest weighted sum or other form of weighted combination may be selected as estimated location 332 of passive acoustic sensor 210. A marker identifying estimated location 332 may be provided in acoustic images 320 which are displayed on display device 116 to a user or operator of acoustic imaging system 100, including for example to a physician performing an interventional procedure using interventional device 200. In some embodiments, thresholding may be employed such that if none of candidate locations 330 provides a good enough match to one, more, or all of the various intra-procedural context-specific information-based constraints, then acoustic imaging system 100 can decline to select and display a marker for an estimated location 332 of passive acoustic sensor 210.

In some embodiments, determining, the exact numerical method for combining the different information sources, as well as the actual values of the weights, may be done via an empirical optimization routine. The optimization may be carried out for example on training data specific to the desired application. Methods based on statistics or machine learning, for example, may be applied to optimize for a metric of accuracy or reliability on this training data.

In some embodiments, a measure of the certainty or uncertainty of the final determined sensor position may be additionally provided. A highly certain final position determination may in turn be used as a stronger prior constraint when computing the sensor position in the next time frame, particularly when incorporating history information. In contrast, a less certain final result could be made to impose a weaker prior constraint on the position estimate in the subsequent frame.

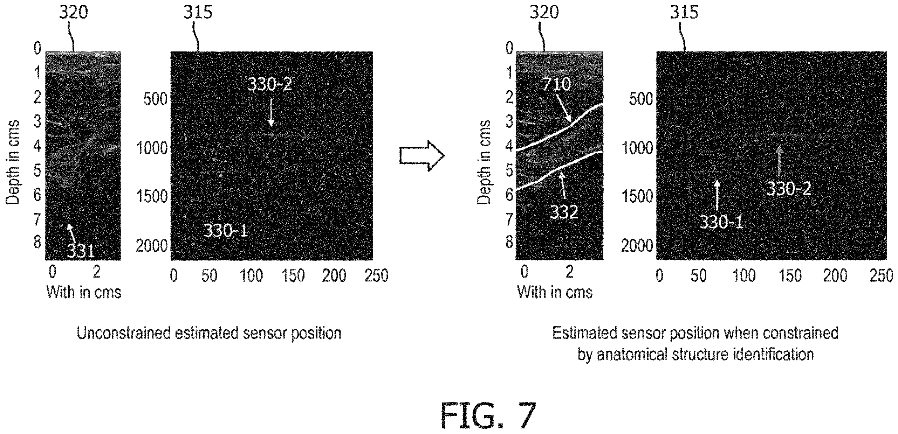

FIGs. 7-9 illustrate in further detail various examples of using intra-procedural context-specific information to ascertain estimated location 332 of passive acoustic sensor 210. Intra-procedural context-specific information may be employed to eliminate candidate locations 330 from consideration for selection as estimated location 332. Intra-procedural context-specific information may be employed to select one of candidate locations 330 which best matches or agrees with the intra-procedural context-specific information as estimated location 332.

FIG. 7 illustrates an example embodiment of a method of improving sensor tracking estimates in interventional acoustic imaging by employing anatomical structure constraints.

The left side of FIG. 7 illustrates a case where no intra-procedural context-specific information-based constraints are employed in estimating the location of passive acoustic sensor 210. Here, image 315 of sensor data shows multiple candidate locations 330-1 and 330-2 for passive acoustic sensor 210. Without further constraints, processor 112 chooses candidate location 330-1 as an incorrect estimated location 331 for passive acoustic sensor 210, for example because its peak intensity is greater than the peak intensity of candidate location 330-2.

The right side of FIG. 7 illustrates a case where an intra-procedural context-specific information-based constraint is employed in estimating the location of passive acoustic sensor 210. In particular, the right side of FIG. 7 illustrates a case where an anatomical structure constraint is employed in selecting one of the candidate locations 330-1 and 330-2 as estimated location 332 of passive acoustic sensor 210. In particular, here is illustrated a case where processor 112 executes a region detection algorithm or segmentation algorithm to identify an anatomical structure 710 (e.g., a blood vessel) where passive acoustic sensor 210 is expected to be located in acoustic images 320. Based on the constraint that passive acoustic sensor 210 should be located within anatomical structure 710, processor 112 selects candidate location 330-2 as estimated location 332.

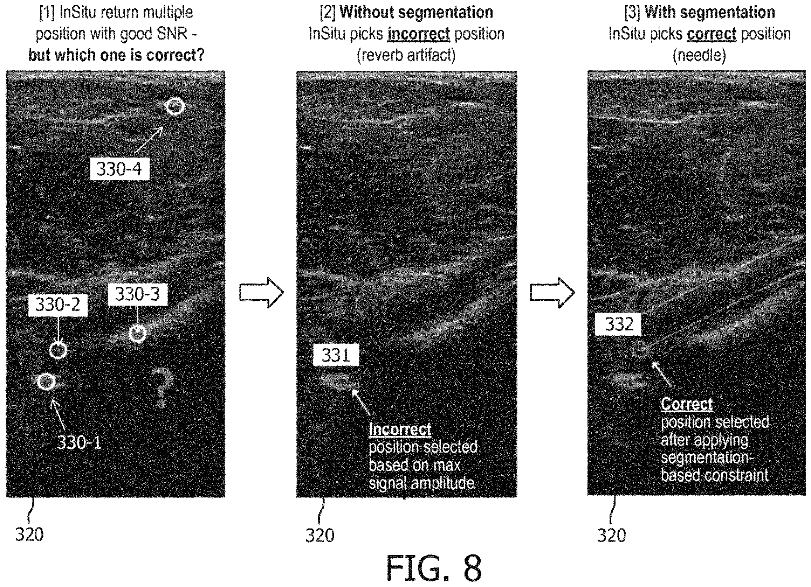

FIG. 8 illustrates one example embodiment of a method of improving sensor tracking estimates in interventional acoustic imaging by employing constraints based on a structure of a device on which the sensor is provided.

For needle interventions, estimated location 332 of passive acoustic sensor 210 has to be on the needle shaft. This constraint can, thus, be used to weed out incorrect candidate locations 330 of passive acoustic sensor 210. In FIG. 8, multiple candidate locations 330-1, 330-2, 330-3 and 330-4 exist for the sensor position (shown scan converted in B-mode space in the leftmost figure). Without employing any context-specific information-based constraints, processor 112 will select incorrect estimated location 331 shown in the central image in FIG. 8. However, when a needle shaft segmentation-based constraint is applied, processor 112 selects the correct estimated position 532 for passive acoustic sensor 210, as shown in the rightmost image in FIG. 8. The different straight lines 810 in the rightmost image in FIG. 8 indicate possible candidates for the shaft of the needle, based on the automated shaft segmentation algorithm used in this example. The correct result is the one where the segmented shaft culminates in the correct estimated position 532 for passive acoustic sensor 210.

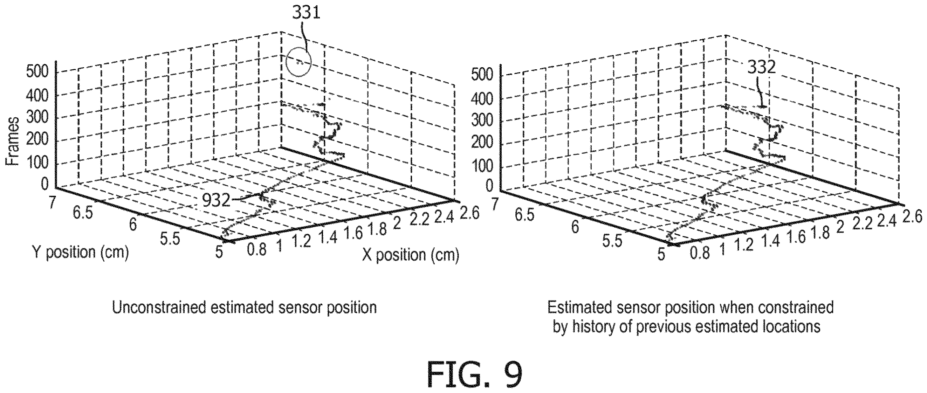

FIG. 9 illustrates one example embodiment of a method of improving sensor tracking estimates in interventional acoustic imaging by employing previous estimated locations of the sensor.

The location of passive acoustic sensor 210 in the current frame or acoustic image 320 cannot be inconsistent with history (i.e., its locations in previous frames or acoustic images 320). Reliance on sensor history can be modelled in different ways. For example, a Kalman filter model framework can be tweaked to either place more weight on the current estimate or rely more on the historical locations. Alternately, principal component analysis (PCA) of all previous estimated locations 332 of passive acoustic sensor 210 can be performed and the first principal component indicates device motion trajectory. In another example, the search space in the current frame or acoustic image 320 can be reduced to a region of interest (ROI) around the estimated location 332 in the previous frame(s) or acoustic image(s) 320. FIG. 9 shows an example where this last method of history-based constraint is used to weed out incorrect sensor location estimates, such as incorrect estimated position 331.

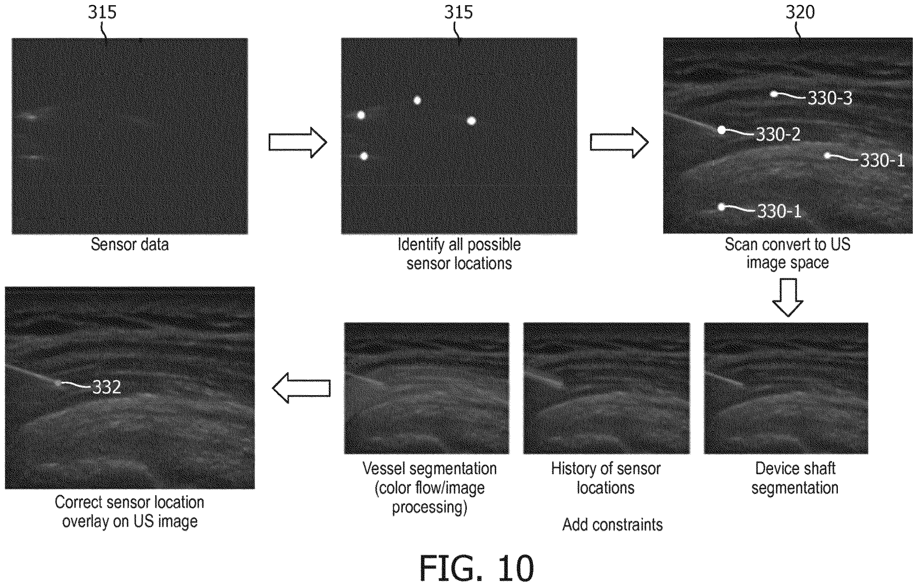

FIG. 10 illustrates graphically an example of improving sensor tracking estimates in interventional acoustic imaging by employing intra-procedural context-specific information, as described above with respect to FIGs. 5-9. In the illustrated example, multiple candidate locations 330-1, 330-2, 330-3 and 330-4 are identified in the sensor data, and then intra-procedural context-specific information is employed to select one of the candidate locations (e.g., candidate location 330-2) as the estimated location of passive acoustic sensor 210. Here, the intra- procedural context-specific information includes anatomical constraint, the known shape of the structure of an interventional device on which passive acoustic sensor 210 is provided, and previous estimated locations of passive acoustic sensor 210.

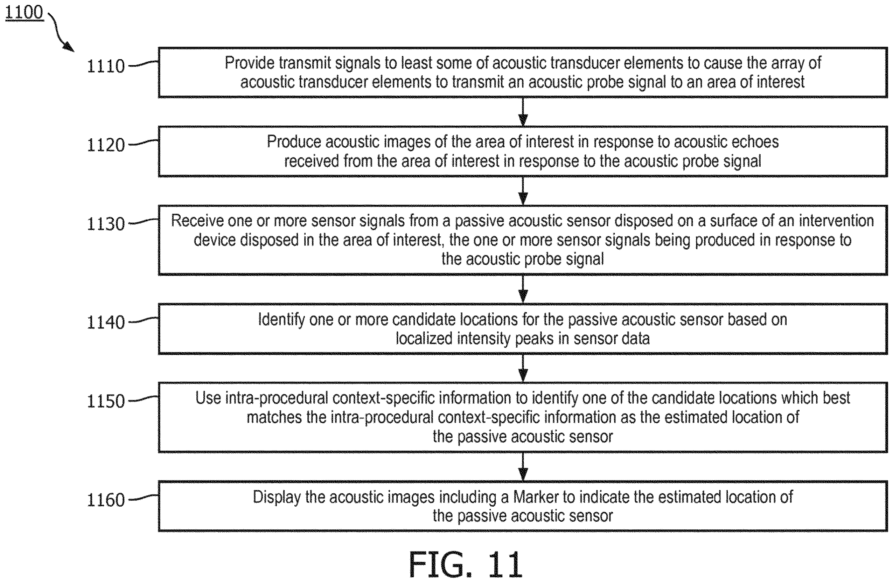

FIG. 11 illustrates a flowchart of an example embodiment of a method of improving sensor tracking estimates in interventional acoustic imaging by employing intra-procedural context-specific information.

An operation 1110 includes providing transmit signals to least some of the acoustic transducer elements of an acoustic probe to cause the array of acoustic transducer elements to transmit an acoustic probe signal to an area of interest.

An operation 1120 includes producing acoustic images of the area of interest in response to acoustic echoes received from the area of interest in response to the acoustic probe signal.

An operation 1130 includes receiving one or more sensor signals from at least one passive acoustic sensor disposed on a surface of an intervention device disposed in the area of interest, the one or more sensor signals being produced in response to the acoustic probe signal.

An operation 1140 includes identifying one or more candidate locations for the passive acoustic sensor based on localized intensity peaks in sensor data.

An operation 1150 includes using intra-procedural context-specific information to identify one of the candidate locations which best matches the intra-procedural context-specific information as the estimated location of the passive acoustic sensor.

An operation 1160 includes displaying the acoustic images including a marker to indicate the estimated location of the passive acoustic sensor in the acoustic image.

It should be understood that the order of various operations in FIG. 11 may be changed or rearranged, and indeed some operations may actually be performed in parallel with one or more other operations. In that sense, FIG. 11 may be better viewed as a numbered list of operations rather than an ordered sequence.

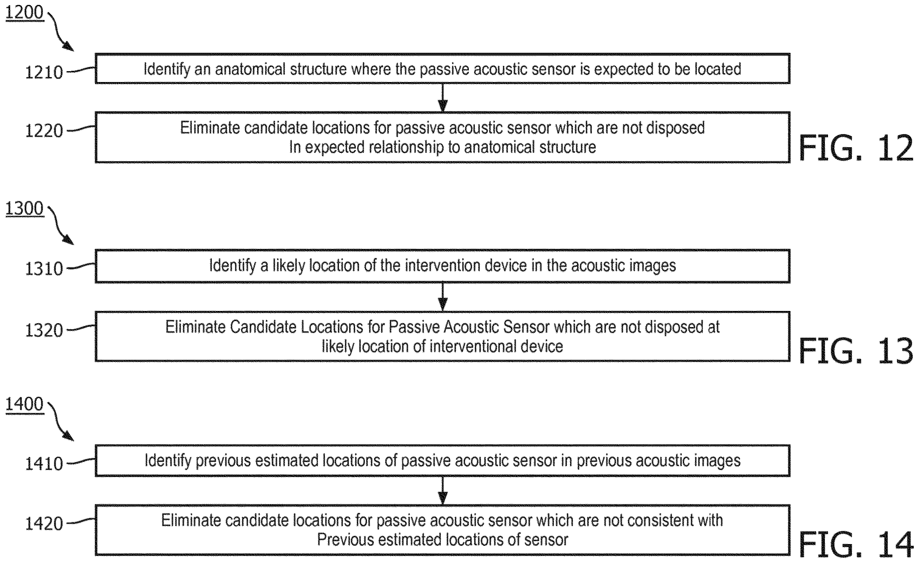

FIG. 12 illustrates a flowchart of an example embodiment of operation 1150 in FIG. 11. In particular, FIG. 12 illustrates a method 1200 of employing anatomical structure constraints to improve sensor tracking estimates in interventional acoustic imaging.

An operation 1210 includes identifying an anatomical structure where the sensor is expected to be located. In some embodiments, this may include executing a region detection algorithm or segmentation algorithm of an acoustic image. In other embodiments, the acoustic imaging instrument is configured to produce color Doppler images of the area of interest in response to one or more receive signals received from the acoustic probe, and the processor is configured to identify the anatomical structure where the sensor is expected to be by identifying blood flow in the color Doppler images.

An operation 1220 includes eliminating candidate locations for the sensor which are not disposed in an expected relationship to the anatomical structure.

FIG. 13 illustrates a flowchart of another example embodiment of operation 1150 in FIG. 11. In particular, FIG. 13 illustrates a method 1300 of employing constraints based on a structure of a device on which a sensor is provided to improve sensor tracking estimates in interventional acoustic imaging.

An operation 1310 includes identifying a likely location of the intervention device in the acoustic images. In some embodiments, this may include executing a region detection algorithm or segmentation algorithm of an acoustic image.

An operation 1320 includes eliminating candidate locations for the passive acoustic sensor which are not disposed at likely location of interventional device.

FIG. 14 illustrates a flowchart of yet another example embodiment of operation 1150 in FIG. 11. In particular, FIG. 14 illustrates a method 1400 of employing previous estimated locations of the sensor to improve sensor tracking estimates in interventional acoustic imaging.

An operation 1410 includes identifying previous estimated locations of the passive acoustic sensor in previous acoustic images.

An operation 1420 includes eliminating candidate locations for the passive acoustic sensor which are not consistent with previous estimated locations of the passive acoustic sensor.

Although not illustrated with a separate flowchart, as explained in detail above, in some embodiments operation 1050 in FIG. 1050 may be performed by employing two or more of the approaches illustrated in FIGs. 12-14 and weighting the results of each algorithm.

A non-exhaustive set of examples of algorithms for using intra-procedural context-specific information to identify one of the candidate locations which best matches the intra-procedural context-specific information as the estimated location of the passive acoustic sensor has been presented here for illustration purposes. Of course other algorithms for using intra-procedural context-specific information to identify one of the candidate locations which best matches the intra-procedural context-specific information as the estimated location of the passive acoustic sensor would become apparent to those skilled in the art after reading the present disclosure, and such algorithms are intended to be encompassed by the broad claims and disclosure presented here.

While preferred embodiments are disclosed in detail herein, many variations are possible which remain within the scope of the invention as defined by the appended claims. Such variations would become clear to one of ordinary skill in the art after inspection of the specification, drawings and claims herein. The invention therefore is not to be restricted except within the scope of the appended claims.

## Claims

1. An acoustic imaging instrument (110) connectable to an acoustic probe (120) having an array of acoustic transducer elements, the acoustic imaging instrument, when connected to the acoustic probe, configured to provide transmit signals to at least some of the acoustic transducer elements to cause the array of acoustic transducer elements to transmit an acoustic probe signal to an area of interest, and further configured to produce acoustic images of the area of interest in response to acoustic echoes received from the area of interest in response to the acoustic probe signal (1110), the acoustic imaging instrument including:
- a receiver interface (118) connectable to an intervention device and configured to receive one or more sensor signals from at least one passive sensor disposed on a surface of the intervention device when the intervention device is connected to the receiver interface and the at least one passive sensor is disposed in the area of interest, the one or more sensor signals having been produced in response to the acoustic probe signal (1130); and
- a processor (112) configured to ascertain, from the one or more sensor signals from the passive sensor, an estimated location of the passive sensor in the area of interest, by:
- identifying one or more candidate locations for the passive sensor based on localized intensity peaks in sensor data produced in response to the one or more sensor signals from the passive sensor (1140), and
- using intra-procedural context-specific information to identify the candidate location of the one or more candidate locations which best matches the intra-procedural context-specific information as the estimated location of the passive sensor (1150),
wherein the processor is further configured to cause a display device (116) to display acoustic images of the area of interest and a marker in the acoustic images to indicate the estimated location of the passive sensor (1160).

2. The acoustic imaging instrument of claim 1, wherein the intra-procedural context-specific information includes at least one of:
- information identifying an anatomical structure where the passive sensor is expected to be located;
- information identifying a likely location of the intervention device in the acoustic images; and
- information identifying previous estimated locations of the passive sensor in previous ones of the acoustic images.

3. The acoustic imaging instrument of claim 2, wherein the intra-procedural context-specific information includes the information identifying the anatomical structure where the passive sensor is expected to be located, and wherein the processor is configured to execute one of a region detection algorithm and a segmentation algorithm to identify the anatomical structure where the passive sensor is expected to be located in the acoustic images.

4. The acoustic imaging instrument of claim 2, wherein the intra-procedural context-specific information includes the information identifying the anatomical structure where the passive sensor is expected to be located, wherein the acoustic imaging instrument is configured to produce color Doppler images of the area of interest in response to one or more receive signals received from the acoustic probe, and wherein the processor is configured to identify the anatomical structure where the passive sensor is expected to be by identifying blood flow in the color Doppler images.

5. The acoustic imaging instrument of claim 2, wherein the intra-procedural context-specific information includes the information identifying the likely location of the intervention device in the acoustic images, and wherein the processor is configured to perform one of a region detection algorithm and a segmentation algorithm to identify the likely location of the intervention device in the acoustic images.

6. The acoustic imaging instrument of claim 2, wherein the intra-procedural context-specific information includes the information identifying the previous estimated locations of the passive sensor in previous ones of the acoustic images, and wherein the processor is configured to employ one of:
- a Kalman filter applied to each one of the one or more candidate locations and the previous estimated locations of the passive sensor;
- a principal component analysis (PCA) of all previous estimated locations of the passive sensor to identify a sensor motion trajectory and compare the sensor motion trajectory to each one of the one or more candidate locations; and
- a region of interest (ROI) spatial filter defined around a previous estimated location of the passive sensor and applied to each one of the one or more candidate locations.

7. The acoustic imaging instrument of claim 1, wherein the intra-procedural context-specific information includes: information identifying an anatomical structure where the passive sensor is expected to be located; information identifying a likely location of the intervention device in the acoustic images; and information identifying previous estimated locations of the passive sensor in previous ones of the acoustic images and wherein identifying the one or more candidate locations for the passive sensor based on the localized intensity peaks in the one or more sensor signals includes:
determining, for each one of the one or more candidate locations, a weighted integration of a match between the candidate location and each of: the information identifying the anatomical structure where the passive sensor is expected to be located; the information identifying the likely location of the intervention device in the acoustic images; and the information identifying the previous estimated locations of the passive sensor in the previous ones of the acoustic images; and
selecting as the estimated location of the passive sensor the candidate location of the one or more candidate locations which has a greatest product of the weighted integration.

8. An acoustic imaging system comprising:
- the acoustic imaging instrument as claimed in any one of the claims 1 to 7;
- an acoustic probe as defined in claim 1;
- a display device as defined in claim 1; and
- an intervention device as defined in claim 1.

9. A computer program comprising computer-readable instructions which, when the computer program is run on a microprocessor of a processor, cause the microprocessor to execute an algorithm to control a system as claimed in claim 8 to perform:
producing acoustic images of an area of interest in response to one or more receive signals received from an acoustic probe in response to acoustic echoes received by the acoustic probe from the area of interest in response to an acoustic probe signal (1120);
receiving one or more sensor signals from a passive sensor disposed on a surface of an intervention device in the area of interest, the one or more sensor signals being produced in response to the acoustic probe signal (1130);
identifying one or more candidate locations for the passive sensor based on localized intensity peaks in sensor data produced in response to the one or more sensor signals from the passive sensor (1140);
using intra-procedural context-specific information to identify the candidate location of the one or more candidate locations which best matches the intra-procedural context-specific information as an estimated location of the passive sensor (1150);
displaying on a display device the acoustic images and a marker in the acoustic images to indicate the estimated location of the passive sensor (1160).

10. A computer program according to claim 9, wherein the intra-procedural context-specific information includes at least one of:
- information identifying an anatomical structure where the passive sensor is expected to be located;
- information identifying a likely location of the intervention device in the acoustic images; and
- information identifying previous estimated locations of the passive sensor in previous ones of the acoustic images.

11. A computer program according to claim 10, wherein the intra-procedural context-specific information includes the information identifying the anatomical structure where the sensor is expected to be located, and wherein said computer program is configured to execute one of a region detection algorithm and a segmentation algorithm to identify the anatomical structure where the passive sensor is expected to be located in the acoustic images.

12. A computer program according to claim 10, wherein the intra-procedural context-specific information includes the information identifying the anatomical structure where the passive sensor is expected to be located, and wherein said computer program is configured to:
produce color Doppler images of the area of interest in response to the one or more receive signals received from the acoustic probe; and
identify the anatomical structure where the passive sensor is expected to be located by identifying blood flow in the color Doppler images.

13. A computer program according to claim 10, wherein the intra-procedural context-specific information includes the information identifying a likely location of the intervention device in the acoustic images, and wherein said computer program is configured to perform one of a region detection algorithm and a segmentation algorithm to identify the likely location of the intervention device in the acoustic images.

14. A computer program according to claim 10, wherein the intra-procedural context-specific information includes the information identifying the previous estimated locations of the passive sensor in previous ones of the acoustic images, and wherein said computer program is configured to:
apply a Kalman filter to each current candidate location and the previous estimated locations of the passive sensor;
perform a principal component analysis (PCA) of all previous locations of the passive sensor to identify sensor motion trajectory, and comparing the sensor motion trajectory to each candidate location; and
apply a region of interest (ROI) spatial filter, defined around an estimated location of the passive sensor in a previous frame, to each one of the one or more candidate locations.

15. A computer program according to claim 9, wherein the intra-procedural context-specific information includes: information identifying an anatomical structure where the passive sensor is expected to be located; information identifying a likely location of the intervention device in the acoustic images; and information identifying previous estimated locations of the passive sensor in previous ones of the acoustic images.

16. A computer program according to claim 15, wherein identifying the one of the candidate locations which best matches the intra-procedural context-specific information as the estimated location of the passive sensor includes:
determining, for each candidate location, a weighted integration of a match between the candidate location and each of: the information identifying the anatomical structure where the sensor is expected to be located; the information identifying the likely location of the intervention device in the acoustic images; and the information identifying the previous estimated locations of the sensor in the previous ones of the acoustic images; and
selecting as the estimated location of the passive sensor a one of the candidate locations which has a greatest weighted combination.

## Patentansprüche

1. Akustisches Bildgebungsinstrument (110), das mit einer akustischen Sonde (120) verbindbar ist, die eine Anordnung von akustischen Wandlerelementen aufweist, wobei das akustische Bildgebungsinstrument, wenn es mit der akustischen Sonde verbunden ist, konfiguriert ist, um Sendesignale an mindestens einige der akustischen Wandlerelemente bereitzustellen, um die Anordnung von akustischen Wandlerelementen zu veranlassen, ein akustisches Sondensignal an einen Bereich von Interesse zu senden, und weiter konfiguriert ist, um akustische Bilder des Bereichs von Interesse als Reaktion auf akustische Echos zu erzeugen, die als Reaktion auf das akustische Sondensignal (1110) aus dem Bereich von Interesse empfangen werden, wobei das akustische Bildgebungsinstrument beinhaltet:
- eine Empfängerschnittstelle (118), die mit einer Interventionsvorrichtung verbindbar ist und konfiguriert ist, um ein oder mehrere Sensorsignale von mindestens einem passiven Sensor zu empfangen, der auf einer Oberfläche der Interventionsvorrichtung angeordnet ist, wenn die Interventionsvorrichtung mit der Empfängerschnittstelle verbunden ist und der mindestens eine passive Sensor in dem Bereich von Interesse angeordnet ist, wobei das eine oder die mehreren Sensorsignale als Reaktion auf das akustische Sondensignal (1130) erzeugt worden sind; und
- einen Prozessor (112), der konfiguriert ist, um aus dem einen oder den mehreren Sensorsignalen des passiven Sensors einen geschätzten Standort des passiven Sensors im Bereich von Interesse zu ermitteln, durch:
- Identifizieren eines oder mehrerer Kandidatenstandorte für den passiven Sensor basierend auf lokalisierten Intensitätsspitzen in Sensordaten, die als Reaktion auf das eine oder die mehreren Sensorsignale aus dem passiven Sensor (1140) erzeugt werden, und
- Verwenden von intraprozeduralen kontextspezifischen Informationen, um den Kandidatenstandort des einen oder der mehreren Kandidatenstandorte, der am besten mit den intraprozeduralen kontextspezifischen Informationen übereinstimmt, als geschätzten Standort des passiven Sensors (1150) zu identifizieren,
wobei der Prozessor weiter konfiguriert ist, um eine Anzeigevorrichtung (116) zu veranlassen, akustische Bilder des Bereichs von Interesse und eine Markierung in den akustischen Bildern anzuzeigen, um den geschätzten Standort des passiven Sensors (1160) anzugeben.

2. Akustisches Bildgebungsinstrument nach Anspruch 1, wobei die intraprozeduralen kontextspezifischen Informationen mindestens eines beinhalten von:
- Informationen, welche eine anatomische Struktur identifizieren, in der erwartet wird, dass sich der passive Sensor befindet;
- Informationen, welche einen wahrscheinlichen Standort der Interventionsvorrichtung in den akustischen Bildern identifizieren; und
- Informationen, welche zuvor geschätzte Standorte des passiven Sensors in vorherigen der akustischen Bilder identifizieren.

3. Akustisches Bildgebungsinstrument nach Anspruch 2, wobei die intraprozeduralen kontextspezifischen Informationen die Informationen beinhalten, welche die anatomische Struktur identifizieren, in der erwartet wird, dass sich der passive Sensor befindet, und wobei der Prozessor konfiguriert ist, um einen von einem Bereichserkennungsalgorithmus und einem Segmentierungsalgorithmus auszuführen, um die anatomische Struktur zu identifizieren, in der erwartet wird, dass sich der passive Sensor in den akustischen Bildern befindet.

4. Akustisches Bildgebungsinstrument nach Anspruch 2, wobei die intraprozeduralen kontextspezifischen Informationen die Informationen beinhalten, welche die anatomische Struktur identifizieren, in der erwartet wird, dass sich der passive Sensor befindet, wobei das akustische Bildgebungsinstrument konfiguriert ist, um Doppler-Farbbilder des Bereichs von Interesse als Reaktion auf ein oder mehrere Empfangssignale zu erzeugen, die von der akustischen Sonde empfangen werden, und wobei der Prozessor konfiguriert ist, um die anatomische Struktur, in der erwartet wird, dass sich der passive Sensor befindet, durch Identifizieren von Blutfluss in den Doppler-Farbbildern zu identifizieren.

5. Akustisches Bildgebungsinstrument nach Anspruch 2, wobei die intraprozeduralen kontextspezifischen Informationen die Informationen beinhalten, die den wahrscheinlichen Standort der Interventionsvorrichtung in den akustischen Bildern identifizieren, und wobei der Prozessor konfiguriert ist, um einen von einem Bereichserkennungsalgorithmus und einem Segmentierungsalgorithmus auszuführen, um den wahrscheinlichen Standort der Interventionsvorrichtung in den akustischen Bildern zu identifizieren.

6. Akustisches Bildgebungsinstrument nach Anspruch 2, wobei die intraprozeduralen kontextspezifischen Informationen die Informationen beinhalten, welche die zuvor geschätzten Standorte des passiven Sensors in vorherigen der akustischen Bilder identifizieren, und wobei der Prozessor konfiguriert ist, um eines von Folgendem einzusetzen:
- einen Kalman-Filter, der auf jeden des einen oder der mehreren Kandidatenstandorte und der zuvor geschätzten Standorte des passiven Sensors angewendet wird;
- eine Hauptkomponentenanalyse (PCA) aller zuvor geschätzten Standorte des passiven Sensors, um eine Sensorbewegungsbahn zu identifizieren, und die Sensorbewegungsbahn mit jedem des einen oder der mehreren Kandidatenstandorte zu vergleichen; und
- einen räumlichen Filter für den Bereich von Interesse (ROI), der um einen zuvor geschätzten Standort des passiven Sensors herum definiert und auf jeden ders einen oder der mehreren Kandidatenstandorte angewendet wird.

7. Akustisches Bildgebungsinstrument nach Anspruch 1, wobei die intraprozeduralen kontextspezifischen Informationen beinhalten: Informationen, welche eine anatomische Struktur identifizieren, in der erwartet wird, dass sich der passive Sensor befindet; Informationen, welche einen wahrscheinlichen Standort der Interventionsvorrichtung in den akustischen Bildern identifizieren; und Informationen, welche zuvor geschätzte Standorte des passiven Sensors in vorherigen der akustischen Bilder identifizieren, und wobei Identifizieren des einen oder der mehreren Kandidatenstandorte für den passiven Sensor basierend auf den lokalisierten Intensitätsspitzen in dem einen oder den mehreren Sensorsignalen beinhaltet:
Bestimmen, für jeden des einen oder der mehreren Kandidatenstandorte, einer gewichteten Integration einer Übereinstimmung zwischen dem Kandidatenstandort und jedem von: den Informationen, welche die anatomische Struktur identifizieren, in der erwartet wird, dass sich der passive Sensor befindet; den Informationen, die den wahrscheinlichen Standort der Interventionsvorrichtung in den akustischen Bildern identifizieren; und den Informationen, welche die zuvor geschätzten Standorte des passiven Sensors in den vorherigen der akustischen Bilder identifizieren; und
Auswählen des Kandidatenstandorts aus dem einen oder den mehreren Kandidatenstandorten als geschätzten Standort des passiven Sensors, der das größte Produkt der gewichteten Integration aufweist.

8. Akustisches Bildgebungssystem, umfassend:
- das akustische Bildgebungsinstrument nach einem der Ansprüche 1 bis 7;
- eine akustische Sonde nach Anspruch 1;
- eine Anzeigevorrichtung nach Anspruch 1; und
- eine Interventionsvorrichtung nach Anspruch 1.

9. Computerprogramm, das computerlesbare Anweisungen umfasst, die, wenn das Computerprogramm auf einem Mikroprozessor eines Prozessors läuft, den Mikroprozessor veranlassen, einen Algorithmus auszuführen, um ein System nach Anspruch 8 zu steuern, um Folgendes durchzuführen:
Erzeugen von akustischen Bildern eines Bereichs von Interesse als Reaktion auf ein oder mehrere Empfangssignale, die von einer akustischen Sonde als Reaktion auf von der akustischen Sonde empfangene akustische Echos aus dem Bereich von Interesse als Reaktion auf ein akustisches Sondensignal (1120) empfangen werden;
Empfangen eines oder mehrerer Sensorsignale von einem passiven Sensor, der auf einer Oberfläche einer Interventionsvorrichtung im Bereich von Interesse angeordnet ist, wobei das eine oder die mehreren Sensorsignale als Reaktion auf das akustische Sondensignal (1130) erzeugt werden;
Identifizieren eines oder mehrerer Kandidatenstandorte für den passiven Sensor basierend auf lokalisierten Intensitätsspitzen in Sensordaten, die als Reaktion auf das eine oder die mehreren Sensorsignale aus dem passiven Sensor (1140) erzeugt werden;
Verwenden von intraprozeduralen kontextspezifischen Informationen, um den Kandidatenstandort des einen oder der mehreren Kandidatenstandorte, der am besten mit den intraprozeduralen kontextspezifischen Informationen übereinstimmt, als einen geschätzten Standort des passiven Sensors (1150) zu identifizieren;
Anzeigen auf einer Anzeigevorrichtung der akustischen Bilder und einer Markierung in den akustischen Bildern, um den geschätzten Standort des passiven Sensors (1160) anzugeben.

10. Computerprogramm nach Anspruch 9, wobei die intraprozeduralen kontextspezifischen Informationen mindestens eines beinhalten von
- Informationen, welche eine anatomische Struktur identifizieren, in der erwartet wird, dass sich der passive Sensor befindet;
- Informationen, welche einen wahrscheinlichen Standort der Interventionsvorrichtung in den akustischen Bildern identifizieren; und
- Informationen, welche zuvor geschätzte Standorte des passiven Sensors in vorherigen der akustischen Bilder identifizieren.

11. Computerprogramm nach Anspruch 10, wobei die intraprozeduralen kontextspezifischen Informationen die Informationen beinhalten, welche die anatomische Struktur identifizieren, in der erwartet wird, dass sich der Sensor befindet, und wobei das Computerprogramm konfiguriert ist, um einen von einem Bereichserkennungsalgorithmus und einem Segmentierungsalgorithmus auszuführen, um die anatomische Struktur zu identifizieren, in der erwartet wird, dass sich der passive Sensor in den akustischen Bildern befindet.

12. Computerprogramm nach Anspruch 10, wobei die intraprozeduralen kontextspezifischen Informationen die Informationen beinhalten, welche die anatomische Struktur identifizieren, in der erwartet wird, dass sich der passive Sensor befindet, und wobei das Computerprogramm konfiguriert ist, um:
Doppler-Farbbilder des Bereichs von Interesse als Reaktion auf das eine oder die mehreren Empfangssignale, die von der akustischen Sonde empfangen werden, zu erzeugen; und
die anatomische Struktur, in der erwartet wird, dass sich der passive Sensor befindet, durch Identifizieren von Blutfluss in den Doppler-Farbbildern zu identifizieren.

13. Computerprogramm nach Anspruch 10, wobei die intraprozeduralen kontextspezifischen Informationen die Informationen beinhalten, die einen wahrscheinlichen Standort der Interventionsvorrichtung in den akustischen Bildern identifizieren, und wobei das Computerprogramm konfiguriert ist, um einen von einem Bereichserkennungsalgorithmus und einem Segmentierungsalgorithmus auszuführen, um den wahrscheinlichen Standort der Interventionsvorrichtung in den akustischen Bildern zu identifizieren.

14. Computerprogramm nach Anspruch 10, wobei die intraprozeduralen kontextspezifischen Informationen die Informationen beinhalten, welche die zuvor geschätzten Standorte des passiven Sensors in vorherigen der akustischen Bilder identifizieren, und wobei das Computerprogramm konfiguriert ist, um:
einen Kalman-Filter auf jeden aktuellen Kandidatenstandort und die zuvor geschätzten Standorte des passiven Sensors anzuwenden;
eine Hauptkomponentenanalyse (PCA) aller vorherigen Standorte des passiven Sensors durchzuführen, um die Sensorbewegungsbahn zu identifizieren, und die Sensorbewegungsbahn mit jedem Kandidatenstandort zu vergleichen; und
einen räumlichen Filter für den Bereich von Interesse (ROI), der um einen zuvor geschätzten Standort des passiven Sensors herum in einem vorherigen Einzelbild definiert wird, auf jeden des einen oder der mehreren Kandidatenstandorte anzuwenden.

15. Computerprogramm nach Anspruch 9, wobei die intraprozeduralen kontextspezifischen Informationen beinhalten: Informationen, welche eine anatomische Struktur identifizieren, in der erwartet wird, dass sich der passive Sensor befindet; Informationen, welche einen wahrscheinlichen Standort der Interventionsvorrichtung in den akustischen Bildern identifizieren; und Informationen, welche zuvor geschätzte Standorte des passiven Sensors in vorherigen der akustischen Bilder identifizieren.

16. Computerprogramm nach Anspruch 15, wobei Identifizieren des einen der Kandidatenstandorte, der am besten mit den intraprozeduralen kontextspezifischen Informationen übereinstimmt, als den geschätzten Standort des passiven Sensors beinhaltet:
Bestimmen, für jeden Kandidatenstandort, einer gewichteten Integration einer Übereinstimmung zwischen dem Kandidatenstandort und jedem von: den Informationen, welche die anatomische Struktur identifizieren, in der erwartet wird, dass sich der Sensor befindet; den Informationen, die den wahrscheinlichen Standort der Interventionsvorrichtung in den akustischen Bildern identifizieren; und den Informationen, welche die zuvor geschätzten Standorte des Sensors in den vorherigen der akustischen Bilder identifizieren; und
Auswählen als den geschätzten Standort des passiven Sensors eines der Kandidatenstandorte, der die größte gewichtete Kombination aufweist.

## Revendications

1. Instrument d'imagerie acoustique (110) pouvant être connecté à une sonde acoustique (120) présentant un réseau d'éléments de transducteur acoustique, l'instrument d'imagerie acoustique, lorsqu'il est connecté à la sonde acoustique, étant configuré pour fournir des signaux de transmission à au moins certains des éléments de transducteur acoustique afin d'amener le réseau d'éléments de transducteur acoustique à transmettre un signal de sonde acoustique à une zone d'intérêt, et étant configuré en outre pour produire des images acoustiques de la zone d'intérêt en réponse à des échos acoustiques reçus depuis la zone d'intérêt en réponse au signal de sonde acoustique (1110), l'instrument d'imagerie acoustique incluant :
- une interface de récepteur (118) pouvant être connectée à un dispositif d'intervention et configurée pour recevoir un ou plusieurs signaux de capteur provenant d'au moins un capteur passif disposé sur une surface du dispositif d'intervention lorsque le dispositif d'intervention est connecté à l'interface de récepteur et le au moins un capteur passif est disposé dans la zone d'intérêt, les un ou plusieurs signaux de capteur ayant été produits en réponse au signal de sonde acoustique (1130) ; et
- un processeur (112) configuré pour déterminer, à partir des un ou plusieurs signaux de capteur provenant du capteur passif, un emplacement estimé du capteur passif dans la zone d'intérêt, par l'intermédiaire des étapes consistant à :
- identifier un ou plusieurs emplacements candidats pour le capteur passif sur la base de pics d'intensité localisés dans des données de capteur produites en réponse aux un ou plusieurs signaux de capteur provenant du capteur passif (1140), et
- utiliser des informations spécifiques à un contexte intra-procédural pour identifier l'emplacement candidat des un ou plusieurs emplacements candidats qui correspond le mieux aux informations spécifiques à un contexte intra-procédure en tant qu'emplacement estimé du capteur passif (1150),
dans lequel le processeur est en outre configuré pour amener un dispositif d'affichage (116) à afficher des images acoustiques de la zone d'intérêt et un marqueur dans les images acoustiques pour indiquer l'emplacement estimé du capteur passif (1160).

2. Instrument d'imagerie acoustique selon la revendication 1, dans lequel les informations spécifiques à un contexte intra-procédural incluent au moins certaines parmi :
- des informations identifiant une structure anatomique où le capteur passif est supposé se trouver ;
- des informations identifiant un emplacement probable du dispositif d'intervention dans les images acoustiques ; et
- des informations identifiant des emplacements estimés précédents du capteur passif dans certaines précédentes des images acoustiques.

3. Instrument d'imagerie acoustique selon la revendication 2, dans lequel les informations spécifiques à un contexte intra-procédural incluent les informations identifiant la structure anatomique où le capteur passif est supposé se trouver, et dans lequel le processeur est configuré pour exécuter l'un d'un algorithme de détection de région et d'un algorithme de segmentation pour identifier la structure anatomique où le capteur passif est supposé se trouver dans les images acoustiques.

4. Instrument d'imagerie acoustique selon la revendication 2, dans lequel les informations spécifiques à un contexte intra-procédural incluent les informations identifiant la structure anatomique où le capteur passif est supposé se trouver, dans lequel l'instrument d'imagerie acoustique est configuré pour produire des images Doppler couleur de la zone d'intérêt en réponse à un ou plusieurs signaux de réception reçus depuis la sonde acoustique, et dans lequel le processeur est configuré pour identifier la structure anatomique où le capteur passif est supposé se trouver en identifiant le flux sanguin dans les images Doppler couleur.

5. Instrument d'imagerie acoustique selon la revendication 2, dans lequel les informations spécifiques à un contexte intra-procédural incluent les informations identifiant l'emplacement probable du dispositif d'intervention dans les images acoustiques, et dans lequel le processeur est configuré pour exécuter l'un d'un algorithme de détection de région et d'un algorithme de segmentation pour identifier l'emplacement probable du dispositif d'intervention dans les images acoustiques.

6. Instrument d'imagerie acoustique selon la revendication 2, dans lequel les informations spécifiques à un contexte intra-procédural incluent les informations identifiant les emplacements estimés précédents du capteur passif dans certaines précédentes des images acoustiques, et dans lequel le processeur est configuré pour utiliser l'un des éléments suivants :
- un filtre de Kalman appliqué à chacun des un ou plusieurs emplacements candidats et aux emplacements estimés précédents du capteur passif ;
- une analyse en composantes principales (PCA) de tous les emplacements estimés précédents du capteur passif pour identifier une trajectoire de mouvement de capteur et comparer la trajectoire de mouvement de capteur à chacun des un ou plusieurs emplacements candidats ; et
- un filtre spatial de région d'intérêt (ROI) défini autour d'un emplacement estimé précédent du capteur passif et appliqué à chacun des un ou plusieurs emplacements candidats.

7. Instrument d'imagerie acoustique selon la revendication 1, dans lequel les informations spécifiques à un contexte intra-procédural incluent : des informations identifiant une structure anatomique où le capteur passif est supposé se trouver ; des informations identifiant un emplacement probable du dispositif d'intervention dans les images acoustiques ; et des informations identifiant des emplacements estimés précédents du capteur passif dans certaines précédentes des images acoustiques, et dans lequel l'identification des un ou plusieurs emplacements candidats pour le capteur passif sur la base des pics d'intensité localisés dans les un ou plusieurs signaux de capteur inclut les étapes consistant à :
déterminer, pour chacun des un ou plusieurs emplacements candidats, une intégration pondérée d'une correspondance entre l'emplacement candidat et chacune parmi : les informations identifiant la structure anatomique où le capteur passif est supposé se trouver ; les informations identifiant l'emplacement probable du dispositif d'intervention dans les images acoustiques ; et les informations identifiant les emplacements estimés précédents du capteur passif dans les certaines précédentes des images acoustiques ; et
sélectionner comme emplacement estimé du capteur passif l'emplacement candidat parmi les un ou plusieurs emplacements candidats présentant le plus grand produit de l'intégration pondérée.

8. Système d'imagerie acoustique, comprenant :
- l'instrument d'imagerie acoustique selon l'une quelconque des revendications 1 à 7 ;
- une sonde acoustique telle que définie dans la revendication 1 ;
- un dispositif d'affichage tel que défini dans la revendication 1 ; et
- un dispositif d'intervention tel que défini dans la revendication 1.

9. Programme informatique comprenant des instructions lisibles par ordinateur qui, lorsque le programme informatique est exécuté sur un microprocesseur d'un processeur, amènent le microprocesseur à exécuter un algorithme pour commander un système selon la revendication 8 pour exécuter les étapes consistant à :
produire des images acoustiques d'une zone d'intérêt en réponse à un ou plusieurs signaux de réception reçus depuis une sonde acoustique en réponse à des échos acoustiques reçus par la sonde acoustique depuis la zone d'intérêt en réponse à un signal de sonde acoustique (1120) ;
recevoir un ou plusieurs signaux de capteur depuis un capteur passif disposé sur une surface d'un dispositif d'intervention dans la zone d'intérêt, les un ou plusieurs signaux de capteur étant produits en réponse au signal de sonde acoustique (1130) ;
identifier un ou plusieurs emplacements candidats pour le capteur passif sur la base de pics d'intensité localisés dans des données de capteur produites en réponse aux un ou plusieurs signaux de capteur provenant du capteur passif (1140) ;
utiliser des informations spécifiques à un contexte intra-procédural pour identifier l'emplacement candidat des un ou plusieurs emplacements candidats qui correspond le mieux aux informations spécifiques à un contexte intra-procédure en tant qu'emplacement estimé du capteur passif (1150) ;
afficher sur un dispositif d'affichage les images acoustiques et un marqueur dans les images acoustiques pour indiquer l'emplacement estimé du capteur passif (1160).

10. Programme informatique selon la revendication 9, dans lequel les informations spécifiques à un contexte intra-procédural incluent au moins certaines parmi :
- des informations identifiant une structure anatomique où le capteur passif est supposé se trouver ;
- des informations identifiant un emplacement probable du dispositif d'intervention dans les images acoustiques ; et
- des informations identifiant des emplacements estimés précédents du capteur passif dans certaines précédentes des images acoustiques.

11. Programme informatique selon la revendication 10, dans lequel les informations spécifiques à un contexte intra-procédural incluent les informations identifiant la structure anatomique où le capteur passif est supposé se trouver, et dans lequel le processeur est configuré pour exécuter l'un d'un algorithme de détection de région et d'un algorithme de segmentation afin d'identifier la structure anatomique où le capteur passif est supposé se trouver dans les images acoustiques.

12. Programme informatique selon la revendication 10, dans lequel les informations spécifiques à un contexte intra-procédural incluent les informations identifiant la structure anatomique où le capteur passif est supposé se trouver, et dans lequel ledit programme informatique est configuré pour effectuer les étapes consistant à :
produire des images Doppler couleur de la zone d'intérêt en réponse aux un ou plusieurs signaux de réception reçus depuis la sonde acoustique ; et
identifier la structure anatomique où le capteur passif est supposé se trouver en identifiant le flux sanguin dans les images Doppler couleur.

13. Programme informatique selon la revendication 10, dans lequel les informations spécifiques à un contexte intra-procédural incluent les informations identifiant un emplacement probable du dispositif d'intervention dans les images acoustiques, et dans lequel ledit programme informatique est configuré pour exécuter l'un d'un algorithme de détection de région et d'un algorithme de segmentation pour identifier l'emplacement probable du dispositif d'intervention dans les images acoustiques.

14. Programme informatique selon la revendication 10, dans lequel les informations spécifiques à un contexte intra-procédural incluent les informations identifiant les emplacements estimés précédents du capteur passif dans certaines précédentes des images acoustiques, et dans lequel ledit programme informatique est configuré pour :
appliquer un filtre de Kalman à chaque emplacement candidat actuel et aux emplacements estimés précédents du capteur passif;
effectuer une analyse en composantes principales (PCA) de tous les emplacements précédents du capteur passif pour identifier la trajectoire de mouvement de capteur, et comparer la trajectoire de mouvement de capteur à chaque emplacement candidat ; et
appliquer un filtre spatial de région d'intérêt (ROI), défini autour d'un emplacement estimé du capteur passif dans une trame précédente, à chacun des un ou plusieurs emplacements candidats.

15. Programme informatique selon la revendication 9, dans lequel les informations spécifiques à un contexte intra-procédural incluent : des informations identifiant une structure anatomique où le capteur passif est supposé se trouver ; des informations identifiant un emplacement probable du dispositif d'intervention dans les images acoustiques ; et des informations identifiant des emplacements estimés précédents du capteur passif dans certaines précédentes des images acoustiques.

16. Programme informatique selon la revendication 15, dans lequel l'identification de celui des emplacements candidats qui correspond le mieux aux informations spécifiques à un contexte intra-procédural en tant qu'emplacement estimé du capteur passif inclut les étapes consistant à :
déterminer, pour chaque emplacement candidat, une intégration pondérée d'une correspondance entre l'emplacement candidat et chacune parmi : les informations identifiant la structure anatomique où le capteur est supposé se trouver ; les informations identifiant l'emplacement probable du dispositif d'intervention dans les images acoustiques ; et les informations identifiant les emplacements estimés précédents du capteur dans les certaines précédentes des images acoustiques ; et
sélectionner comme emplacement estimé du capteur passif l'un des emplacements candidats qui possède la combinaison pondérée la plus élevée.
